(19) Europäisches Patentamt
European Patent Office
Office européen des brevets

(11) Veröffentlichungsnummer: **0 499 831 A2**

(12) # EUROPÄISCHE PATENTANMELDUNG

(21) Anmeldenummer: **92101208.4**

(22) Anmeldetag: **25.01.92**

(51) Int. Cl.5: **A61K 37/64**, //(A61K37/64, 31:42),(A61K37/64,31:535), (A61K37/64,31:53),(A61K37/64, 31:54),(A61K37/64,31:445)

(30) Priorität: **20.02.91 DE 4105191**

(43) Veröffentlichungstag der Anmeldung: **26.08.92 Patentblatt 92/35**

(84) Benannte Vertragsstaaten: **AT BE CH DE DK ES FR GB GR IT LI NL PT SE**

(71) Anmelder: **CASSELLA Aktiengesellschaft**
**Hanauer Landstrasse 526**
**W-6000 Frankfurt am Main 60(DE)**

(72) Erfinder: **Just, Melitta, Dr.**
**Theodor-Heuss-Strasse 80**
**W-6070 Langen 2(DE)**
Erfinder: **Schönafinger, Karl, Dr.**
**Holunderweg 8**
**W-8755 Alzenau(DE)**

(74) Vertreter: **Urbach, Hans-Georg, Dr. et al**
**CASSELLA AKTIENGESELLSCHAFT**
**Patentabteilung Hanauer Landstrasse 526**
**W-6000 Frankfurt am Main 60(DE)**

(54) **Wirkstoffkombination aus einem Sydnonimin und einem Hirudin.**

(57) Die vorliegende Erfindung betrifft eine Wirkstoffkombination aus einem Sydnonimin und einem Hirudin als antithrombotisches Mittel.

EP 0 499 831 A2

Die vorliegende Erfindung betrifft eine Wirkstoffkombination aus einem Sydnonimin und einem Hirudin und ihre Verwendung.

Sydnonimine sind Verbindungen, deren Pharmakologie schon lange bekannt ist (Chemie in unserer Zeit, 13. Jahrgang, Seite 51 (1984)), wobei neben der vasodilatierenden insbesondere auch die antithrombotischen Wirkungen beschrieben sind (J. Cardiovasc. Pharmacol. 1989, 14 (Suppl. 11), Seite 129). Sydnonimine können als Mittel zur Vorbeugung und Behandlung von Erkrankungen des Herz-Kreislauf-Systems eingesetzt werden und sind als solche im Handel.

Hirudin ist ein Polypeptid, das aus dem medizinischen Blutegel (Hirudo medicinalis) isoliert werden kann und das als Thrombininhibitor bekannt ist (Die Pharmazie 36, 653 (1981)). In neuerer Zeit können durch gentechnologische Verfahren ausreichende Mengen an recombinantem Hirudin (r-Hirudin) bereitgestellt werden (Münch.med. Wschr. 127, 16 (1985)). Aber auch eine große Zahl an synthetischen Hirudinen ist bekannt.

Es wurde nun überraschenderweise gefunden, daß sich die antithrombotischen Wirkungen von Sydnoniminen und Hirudinen gegenseitig verstärken.

Die vorliegende Erfindung betrifft demnach eine Wirkstoffkombination aus einem Sydnonimin und einem Hirudin.

Unter dem Begriff Sydnonimin werden alle pharmakologisch wirksamen Sydnonimine verstanden. Insbesondere sind dies die von den folgenden Veröffentlichungen umfaßten Sydnonimine: DE-OS-1620501; DE-OS-1670127; DE-OS-1695897; EP-A-23343; EP-A-59356; EP-A-76952; EP-A-210474; EP-A-276710; EP-A-312773; EP-A-324408; EP-A-346684; EP-A-346694; EP-A-367036; EP-A-406659 und EP-A-406661.

Bevorzugte Sydnonimine sind solche der allgemeinen Formel I

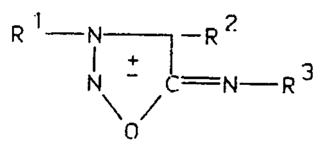

(I)

oder ein pharmakologisch annehmbares Salz davon, wobei $R^1$ eine Aminogruppe der Formel

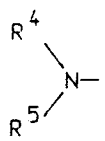 ; 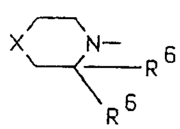 ; 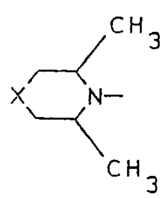 ; 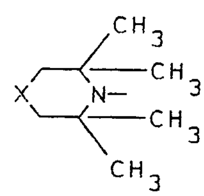 ;

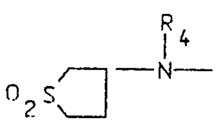 oder 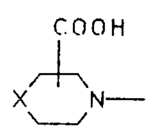

bedeutet;

- $R^2$ Wasserstoff, Alkyl, Cycloalkyl, Arylalkyl, Aryl-X-Alkyl, Alkoxyalkyl, Alkylthioalkyl oder Alkenylthioalkyl bedeutet;
- $R^3$ Wasserstoff oder die Gruppe $-COR^7$ bedeutet;
- $R^4$ Alkyl, Cycloalkyl, Alkyl-X-Alkyl, Arylalkyl, Hydroxyalkyl bedeutet;
- $R^5$ Wasserstoff bedeutet oder eine der Bedeutungen von $R^4$ hat;
- $R^6$ Wasserstoff oder Methyl bedeutet;
- $R^7$ Aryl, durch 1 bis 3 Halogenatome und/oder 1 bis 3 Alkylreste und/oder 1 bis 3 Alkoxyreste und/oder 1 oder 2 Nitrogruppen mono-, di- oder trisubstituierten Arylrest oder $OR^2$ bedeutet oder eine der Bedeutungen von $R^2$ hat;
- X $NR_4$, $NSO_2R^8$, $NCO_2$Alkyl, $S(O)_n$, O, $CH_2$ oder eine einfache Bindung bedeutet;
- n 0, 1 oder 2 ist und
- $R^8$ Alkyl, Aryl, Alkylaryl oder Dialkylamino bedeutet.

Alkylreste und Alkenylreste können geradkettig oder verzweigt sein und haben bevorzugt 1 bis 6 C-Atome, besonders bevorzugt 1 - 4 C-Atome. Dies gilt auch, wenn sie in Verbindung mit anderen Gruppen, z. B. als Alkoxy, Arylalkyl usw. vorliegen.
Beispiele für Alkylreste sind Methyl, Ethyl, Propyl, Isopropyl, Butyl, sek.-Butyl und tert.-Butyl.
Cycloalkyl hat bevorzugt 5 bis 7 C-Atome und bedeutet besonders bevorzugt Cyclopentyl und Cyclohexyl.
Aryl hat bevorzugt 6 bis 10 C-Atome und bedeutet besonders bevorzugt $\alpha$- oder $\beta$-Naphthyl oder Phenyl.
Arylalkyl ist bevorzugt Benzyl und Phenylethyl.
Aryl-X-alkyl ist bevorzugt Phenoxymethyl und Phenoxyethyl.
Die für $R^7$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht. Als für $R^7$ stehende Arylreste sind insbesondere zu nennen: Methylphenyl (= Tolyl), Nitrophenyl und Chlorophenyl, insbesondere 4-Nitrophenyl und 4-Chlorophenyl.
Bevorzugte Reste $R^1$ sind Morpholino, 3,3-Dimethyl-thiomorpholino, cis-2,6-Dimethylpiperidino und 1,1-Dioxo-3,3-dimethyl-thiomorpholino.
$R^2$ bedeutet bevorzugt Wasserstoff.
$R^3$ bedeutet bevorzugt Wasserstoff, Ethoxycarbonyl,
Propionyl oder p-Anisoyl.

Die anspruchsgemäßen Sydnonimine bilden mit anorganischen oder organischen Säuren Säureadditionssalze. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Pharmakologisch annehmbare Säureadditonssalze werden bevorzugt. Besonders bevorzugt sind die Hydrochloride.

Beispiele für anspruchsgemäße Sydnonimine sind:
N-Ethoxycarbonyl-3-morpholino-sydnonimin (Molsidomin); 3-Morpholino-sydnonimin-hydrochlorid (SIN-1); N-(4-Methoxybenzoyl)-3-(cis-2,6-dimethylpiperidino)-sydnonimin; N-Propionyl-3-(3,3-dimethyl-thiomorpholino)sydnonimin; 3-(1,1-Dioxo-3,3-dimethyl-thiomorpholino)-sydnonimin-hydrochlorid; 3-(3,3-Dimethyl-thiomorpholino)-sydnonimin-hydrochlorid; 3-(cis-2,6-Dimethylpiperidino)-sydnonimin-hydrochlorid; 3-(tert.-Butyl-2-hydroxyethyl-amino)-sydnonimin-hydrochlorid; 3-(tert.-Butyl-(2-diisopropylamino-ethyl)-amino)-sydnonimin-dihydrochlorid; 3-(tert.-Butyl-(2-morpholinoethyl)-amino)-sydnonimin-hydrochlorid; 3-(3,3-Dimethylmorpholino)-sydnonimin-hydrochlorid; 3-(2,2-Dimethylpiperidino)-sydnonimin-hydrochlorid; 3-(4-Isopropyl-2,2-dimethyl-piperazino)sydnonimin-dihydrochlorid; N-p-Anisoyl-3-(4-isopropyl-2,2-dimethyl-piperazino)-sydnonimin-dihydrochlorid; 3-(2,2,6,6-Tetramethyl-4-isopropyl-piperazino)sydnonimin-dihydrochlorid; 3-(2,6-Dimethylpiperidino)-4-hexylsydnonimin-hydrochlorid; 4-Benzyl-3-(2,6-dimethylpiperidino)-sydnonimin-hydrochlorid; 3-(2,6-Dimethylpiperidino)-4-(2-phenylethyl)-sydnonimin-hydrochlorid; 3-(2,6-Dimethylpiperidino)-4-phenylthiomethyl-sydnonimin-hydrochlorid; N-Benzoyl-4-benzylthiomethyl-3-(2,6-dimethylpiperidino)-sydnonimin; 3-(2,6-Dimethylpiperidino)-4-phenoxymethylsydnonimin-hydrochlorid; 3-(2-Hydroxyethylamino)-sydnonimin-hydrochlorid; 3-Ethylamino-sydnonimin-hydrochlorid; 3-Cyclohexylaminosydnonimin-hydrochlorid; 3-(2-Hydroxycyclohexyl-amino)-sydnonimin-hydrochlorid; 3-(3,3-Dimethyl-1,4-tetrahydrothiazin-1,1-dioxo-4-yl)-sydnonimin-hydrochlorid; 3-(3,3-Dimethyl-perhydro-1-oxo-1,4-thiazin-4-yl)sydnonimin-hydrochlorid; N-p-Anisoyl-3-dicyclohexylamino-sydnonimin; N-Pivaloyl-3-dicyclohexylamino-sydnonimin; 3-(2-Carboxy-piperidino)-sydnonimin-hydrochlorid; 3-(2,2-Dimethyl-4-methansulfonyl-piperazino)-sydnonimin-hydrochlorid; 3-(2,2-Dimethyl-4-toluolsulfonyl-piperazino)-sydnonimin-hydrochlorid; 3-(2,2-Dimethyl-4-dimethylaminosulfonyl-piperazino)sydnonimin-hydrochlorid; N-Isobutyroyl-3-(2,2-dimethyl-4-methansulfonyl-piperazino)-sydnonimin; 3-(N-tert.-Butyl-N-methylamino)sydnonimin-hydrochlorid; 3-(N-tert.-Butyl-N-butylamino)sydnonimin-hydrochlorid.

Unter dem Begriff Hirudine werden alle pharmakologisch wirksamen Hirudine verstanden. Diese können natürlichen oder synthetischen, aber auch gentechnischen Ursprungs sein. Über die wirksame Aminosäuresequenz hinaus N- oder C-terminal verlängerte Verbindungen werden ebenso umfaßt wie pharmakologisch wirksame Teilsequenzen und Bruchstücke. Im Vergleich zur natürlichen Sequenz können auch einzelne oder mehrere Aminosäuren modifiziert oder gegen andere Aminosäuren und zwar natürlich vorkommende ebenso wie nicht natürlich vorkommende, ausgetauscht sein.

Insbesondere fallen unter den Begriff Hirudine die in folgenden Veröffentlichungen genannten Verbindungen:
DE-A-3445532; EP-A-142860; EP-A-158564; EP-A-158986; EP-A-168342; EP-A-171024; EP-A-173619; EP-A-193175; EP-A-200655; EP-A-209061; EP-A-225633; EP-A-227938; EP-A-236330; EP-A-252854; EP-A-273800; EP-A-324712; EP-A-345616; EP-A-364942.

Besonders bevorzugte Hirudine sind recombinante Desulfato-Hirudine (r-Hirudine), die aus E.coli, Hefe oder B.subtilis gewonnen werden und sich in 2 Aminosäuren vom natürlichen Hirudin unterscheiden. Ein solches r-Hirudin ist beispielsweise HBW 023 (r-desulfato-$Ile^1$ - $Thr^2$ -$Hirudin^{1-65}$; Drugs of the Future 15, 267-280 (1980)).

In der erfindungsgemäßen Wirkstoffkombination kann das Gewichtsverhältnis zwischen Sydnonimin und Hirudin in weiten Bereichen schwanken.

Insbesondere ist das Gewichtsverhältnis Sydnonimin : Hirudin 1 : (0,025 bis 40), bevorzugt 1 : (0,1 bis 10).

Die erfindungsgemäße Wirkstoffkombination kann durch Mischen der Einzelwirkstoffe in den genannten Gewichtsverhältnissen hergestellt werden.

Es ist normalerweise zweckmäßig, die Wirkstoffkombination in Form einer pharmazeutischen Zubereitung zu verabreichen. Diese Zubereitungen können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Zubereitungen können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Zubereitungen können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch jeweils zwei oder mehrere Sydnonimine und Hirudine und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

In einer besonderen Ausführungsform der vorliegenden Erfindung wird die erfindungsgemäße Wirkstoffkombination nicht in Form einer Mischung, d.h. in einer pharmazeutischen Zubereitung, sondern getrennt voneinander, d.h. in zwei getrennten pharmazeutischen Zubereitungen, gleichzeitig oder kurz hintereinander verabreicht.

In diesem Fall enthalten die pharmazeutischen Zubereitungen entweder Sydnonimin oder Hirudin, entsprechen aber ansonsten den oben beschriebenen Zubereitungen.

In einer weiteren Ausführungsform der vorliegenden Erfindung wird eine Zubereitung, die Sydnonimin und Hirudin enthält und gleichzeitig oder kurz danach eine Zubereitung, die Sydnonimin oder Hirudin enthält, verabreicht. Auch in diesem Fall entsprechen die Zubereitungen den oben beschriebenen.

Die erfindungsgemäßen Wirkstoffkombinationen können am Menschen bei der Bekämpfung bzw. Vorbeugung von Erkrankungen des cardiovaskulären Systems verwendet werden, insbesondere als antithrombotisches Heilmittel.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, Wirkstoffkombination angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Überraschend verstärken sich in den erfindungsgemäßen Wirkstoffkombinationen die antithrombotischen Wirkungen von Sydnonimin und Hirudin gegenseitig. Somit läßt sich bereits bei Dosierungen, bei denen weder Sydnonimin noch Hirudin für sich allein wirken, eine sehr starke Wirkung erzielen.

Diese Wirkung wurde in folgendem Test ermittelt:

Bei männlichen, ca. 4 Monate alten Hausschweinen wurde eine Coronarthrombose nach der Methode von Folts et al (Circulation 1976, 54; 365 - 70) erzeugt. Bei den tief narkotisierten Tieren wurde der Thorax eröffnet; die linke absteigende Coronararterie wurde freipräpariert und mit einem elektromagnetischen Blutströmungsmeßkopf versehen. Distal des Meßkopfes wurde das Coronargefäß durch kurzes (1 s) Quetschen mit einer Gefäßklemme geschädigt. Auf diese Stelle wurde ein Plastikkonstriktor (innerer Durchmesser 1.7 - 2.8 mm, Länge 2 mm) gesetzt, der das Gefäß stenosiert. Diese Versuchsbedingungen führten zum Auftreten regelmäßiger zyklischer Blutströmungsveränderungen (CFV's), verursacht durch die wiederholte Bildung und Embolisierung von Thromben an der geschädigten stenosierten Stelle. Die Thromben wurden durch Bestimmung der Frequenz (Anzahl pro 60 min) der zyklischen Blutströmungsveränderungen quantifiziert. Nachdem regelmäßige Zyklen für 60 min aufgetreten waren, erhielten die Tiere eine der folgenden Substanzen:

Gruppe 1 (n = 6): N-Ethoxycarbonyl-3-morpholino-sydnonimin (Molsidomin), 0.1 mg/kg intravenös als Bolusinjektion;

Gruppe 2 (n = 6): recombinantes Desulfato-Hirudin (r-Hirudin) in einer Gesamtdosis von 0.2 mg/kg, verteilt als intravenöse Bolusinjektion von 0.1 mg/kg, gefolgt von einer intravenösen Dauerinfusion von 16.67 µg/kg min (≙ 1 mg/kg 60 min);

Gruppe 3 (n = 6): Molsidomin + r-Hirudin, d.h. 0.1 mg/kg Molsidomin i.v. zusammen mit 0.1 mg/kg r-Hirudin i.v., gefolgt von 0.1 mg/kg•60 min r-Hirudin - Infusion.

In Vorversuchen wurden diese Einzeldosierungen von Molsidomin und r-Hirudin als gerade nicht antithrombotisch wirkend ermittelt.

Der Versuch wurde 1 Stunde nach Ende der Infusion bzw. 2 Stunden nach Bolusinjektion der Substanzen beendet. Die Frequenzen der zyklischen Blutströmungsveränderungen in der Stunde vor und in der ersten und zweiten Stunde nach Injektion der Prüfsubstanzen wurden für jede Versuchsgruppe gemittelt. Die mittleren Frequenzen der Zyklen vor und nach Substanzverabreichung wurden statistisch miteinander verglichen (Student-t-Test für verbundene Stichproben). Ein p - Wert von < 0.05 galt als signifikanter Unterschied. Die Ergebnisse sind der folgenden Tabelle zu entnehmen:

| Versuchsgruppe | Anzahl thrombotischer Flowzyklen (n/60 min) | | |
|---|---|---|---|
| | 1. Stunde | 1. Stunde | 2. Stunde |
| | Vorphase | nach Substanzinjektion | |
| 1 (Molsidomin) | 16 ± 2 | 19 ± 3 | # |
| 2 (r-Hirudin) | 12 ± 1 | 11 ± 2 | 12 ± 1 |
| 3 | 12 ± 1 | 8 ± 2 | 2 ± 2* |

\# keine Wirkung; Zyklenzahl nicht bestimmbar, da z.T. Dauerocclusion auftrat

* p < 0.01 im Vergleich zur Vorphase

Bei allen Tieren wurde zusätzlich der systolische und diastolische periphere arterielle Blutdruck (mm Hg) gemessen. In der Gruppe 2 veränderte die r-Hirudin-Infusion den Blutdruck nicht. Molsidomin (Gruppe 1) senkte in der verabreichten Dosierung den systolischen Blutdruck um maximal 18 ± 5 mm Hg ($p < 0.05$), den diastolischen Blutdruck nicht signifikant um 10 ± 5 mm Hg. In der Kombination mit r-Hirudin (Gruppe 3) senkte Molsidomin den systolischen Blutdruck um 21 ± 4 ($p < 0.05$) und den diastolischen Blutdruck nicht signifikant um 11 ± 3 mm Hg. Diese Blutdruck senkende Wirkung von Molsidomin war in beiden Versuchsgruppen gleich.

Die Ergebnisse zeigen, daß die erfindungsgemäße Wirkstoffkombination in diesen Dosierungen zu einer nahezu totalen Hemmung der experimentellen Thromben führt. Die antithrombotischen Wirkungen dieser beiden Substanzen mit unterschiedlichem Wirkungsmechanismus potenzieren sich demnach ohne zu einer Steigerung der hämodynamischen Wirkung zu führen.

Der Vorteil der erfindungsgemäßen Wirkstoffkombination liegt darin, daß beide Substanzen niedrig dosiert werden können. Dadurch sinkt das Nebenwirkungspotential und die Kombination kann auch bei solchen Patienten eingesetzt werden, deren Kreislaufsituation eine Blutdrucksenkung nicht zuläßt. Ein weiterer Vorteil der erfindungsgemäßen Kombination besteht darin, daß durch die gleichzeitige Beeinflussung zweier unabhängiger Thrombosemechanismen eine besonders effektive Behandlung und Prophylaxe thrombo-embolischer Erkrankungen möglich ist. Einerseits wird durch das Hirudin die Wirkung von Thrombin aufgehoben. Dadurch wird die Fibrinbildung und die Plättchenaktivierung gehemmt. Thrombin gilt als wichtigster Plättchenaktivator auch in Arterien (Proc.Natl.Acad.Sci. USA 1988; 85: 3184-88). Andererseits

aktiviert das Sydnonimin eine der beiden endogenen Gegenregulationen der Thrombozytenstimulierung durch NO-vermittelte Erhöhung des zyklischen Guanosinmonophosphats. Dies führt zur Hemmung der Thrombozytenadhäsion und -aggregation (Circulation 1989; 79: 657-65). Beide Mechanismen zusammen potenzieren einander in der antithrombotischen Wirkung.

Die folgenden Beispiele erläutern die erfindungsgemäße Wirkstoffkombination:

Beispiel 1

N-Ethoxycarbonyl-3-morpholino-sydnonimin (Molsidomin) und r-Hirudin im Gewichtsverhältnis 1 : 2.

Beispiel 2

3-Morpholino-sydnonimin-hydrochlorid (SIN-1) und r-Hirudin im Gewichtsverhältnis 1 : 2.

Beispiel 3

N-(4-Methoxybenzoyl)-3-(cis-2,6-dimethylpiperidino)-sydnonimin und r-Hirudin im Gewichtsverhältnis 5 : 2.

Beispiel 4

N-Propionyl-3-(3,3-dimethylthiomorpholino)-sydnonimin und r-Hirudin im Gewichtsverhältnis 1 : 2.

Beispiel 5

3-(3,3-dimethyl-1,1-dioxo-thiomorpholino)-sydnonimin-hydrochlorid und r-Hirudin im Gewichtsverhältnis 1 : 1.

Beispiel 6

3-(3,3-Dimethyl-thiomorpholino)-sydnonimin-hydrochlorid und r-Hirudin im Gewichtsverhältnis 1 : 3.

Beipiel 7

3-(cis-2,6-Dimethylpiperidino)-sydnonimin-hydrochlorid und r-Hirudin im Gewichtsverhältnis 1 : 1.

## Patentansprüche

1. Wirkstoffkombination aus einem Sydnonimin und einem Hirudin.

2. Wirkstoffkombination nach Anspruch 1, dadurch gekennzeichnet, daß sie ein Sydnonimin der allgemeinen Formel I

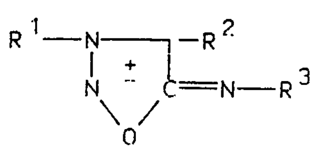

(I)

oder ein pharmakologisch annehmbares Salz davon, wobei

$R^1$ eine Aminogruppe der Formel

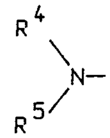 ; 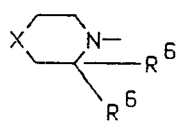 ; 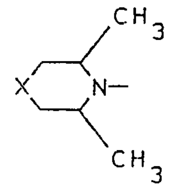 ; 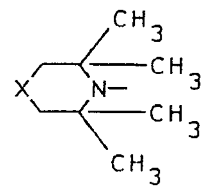 ;

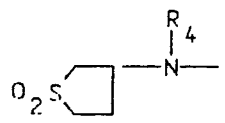 oder 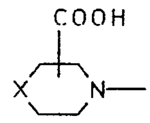

bedeutet;

$R^2$ Wasserstoff, Alkyl, Cycloalkyl, Arylalkyl, Aryl-x-Alkyl, Alkoxyalkyl, Alkylthioalkyl oder Alkenylthioalkyl bedeutet;

$R^3$ Wasserstoff oder die Gruppe $-COR^7$ bedeutet;

$R^4$ Alkyl, Cycloalkyl, Alkyl-X-Alkyl, Arylalkyl oder Hydroxyalkyl bedeutet,

$R^5$ Wasserstoff bedeutet oder eine der Bedeutungen von $R^4$ hat;

$R^6$ Wasserstoff oder Methyl bedeutet;

$R^7$ Aryl, durch 1 bis 3 Halogenatome und/oder 1 bis 3 Alkylreste und/oder 1 bis 3 Alkoxyreste und/oder 1 oder 2 Nitrogruppen mono-, di- oder trisubstituierten Arylrest oder $OR^2$ bedeutet oder eine der Bedeutungen von $R^2$ hat;

X $NR_4$, $NSO_2R^8$, $NCO_2$Alkyl, $S(O)_n$, O, $CH_2$ oder eine einfache Bindung bedeutet;

n 0, 1 oder 2 ist und

$R^8$ Alkyl, Aryl, Alkylaryl oder Dialkylamino bedeutet, enthält.

**3.** Wirkstoffkombination nach Anspruch 2, dadurch gekennzeichnet, daß $R^1$ Morpholino, 3,3-Dimethylthiomorpholino, cis-2,6-Dimethylpiperidino oder 1,1-Dioxo-3,3-dimethylthiomorpholino bedeutet.

**4.** Wirkstoffkombination nach Anspruch 2 und/oder 3, dadurch gekennzeichnet, daß $R^2$ Wasserstoff bedeutet.

**5.** Wirkstoffkombination nach einem oder mehreren der Ansprüche 2 bis 4, dadurch gekennzeichnet, daß $R^3$ Wasserstoff, Ethoxycarbonyl, Propionyl oder p-Anisoyl bedeutet.

**6.** Wirkstoffkombination nach einem oder mehreren der Ansprüche 1 bis 5, dadurch gekennzeichnet, daß als Hirudin ein recombinantes Desulfato-Hirudin (r-Hirudin) verwendet wird.

**7.** Verwendung einer Wirkstoffkombination gemäß einem oder mehreren der Ansprüche 1 bis 6 zur Herstellung von antithrombotischen Mitteln.

**8.** Pharmazeutisches Präparat, dadurch gekennzeichnet, daß es eine Wirkstoffkombination gemäß einem oder mehreren der Ansprüche 1 bis 6 zusammen mit pharmazeutisch annehmbaren Träger- und Zusatzstoffen, gegebenenfalls noch einem oder mehreren anderen pharmakologischen Wirkstoffen und gegebenenfalls weiteren therapeutisch wirksamen Stoffen enthält.

**Patentansprüche für folgende Vertragsstaaten : ES, GR**

**1.** Verfahren zur Herstellung einer Wirkstoffkombination aus einem Sydnonimin und einem Hirudin, dadurch gekennzeichnet, daß Sydnonimin und Hirudin gemischt werden.

**2.** Verfahren nach Anspruch 1, dadurch gekennzeichnet, daß ein Sydnonimin der allgemeinen Formel I

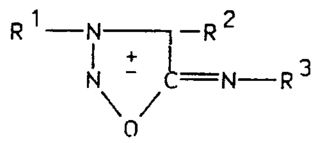

( I )

oder ein pharmakologisch annehmbares Salz davon, wobei

$R^1$ eine Aminogruppe der Formel

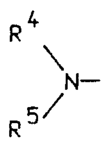 ; 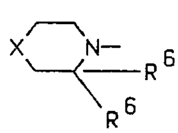 ; 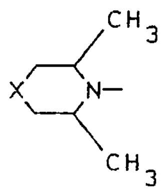 ; 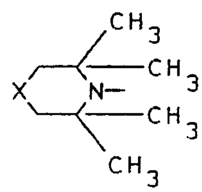 ;

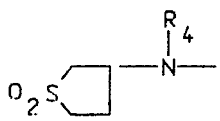 oder 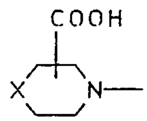

bedeutet;

$R^2$ Wasserstoff, Alkyl, Cycloalkyl, Arylalkyl, Aryl-x-Alkyl, Alkoxyalkyl, Alkylthioalkyl oder Alkenylthioalkyl bedeutet;

$R^3$ Wasserstoff oder die Gruppe $-COR^7$ bedeutet;

$R^4$ Alkyl, Cycloalkyl, Alkyl-X-Alkyl, Arylalkyl oder Hydroxyalkyl bedeutet,

$R^5$ Wasserstoff bedeutet oder eine der Bedeutungen von $R^4$ hat;

$R^6$ Wasserstoff oder Methyl bedeutet;

$R^7$ Aryl, durch 1 bis 3 Halogenatome und/oder 1 bis 3 Alkylreste und/oder 1 bis 3 Alkoxyreste und/oder 1 oder 2 Nitrogruppen mono-, di- oder trisubstituierten Arylrest oder $OR^2$ bedeutet oder eine der Bedeutungen von $R^2$ hat;

X $NR_4$, $NSO_2R^8$, $NCO_2$Alkyl, $S(O)_n$, O, $CH_2$ oder eine einfache Bindung bedeutet;

n 0, 1 oder 2 ist und

$R^8$ Alkyl, Aryl, Alkylaryl oder Dialkylamino bedeutet, eingesetzt wird.

**3.** Verfahren nach Anspruch 2, dadurch gekennzeichnet, daß $R^1$ Morpholino, 3,3-Dimethyl-thiomorpholino, cis-2,6-Dimethylpiperidino oder 1,1-Dioxo-3,3-dimethylthiomorpholino bedeutet.

**4.** Verfahren nach Anspruch 2 und/oder 3, dadurch gekennzeichnet, daß $R^2$ Wasserstoff bedeutet.

**5.** Verfahren nach einem oder mehreren der Ansprüche 2 bis 4, dadurch gekennzeichnet, daß $R^3$ Wasserstoff, Ethoxycarbonyl, Propionyl oder p-Anisoyl bedeutet.

**6.** Verfahren nach einem oder mehreren der Ansprüche 1 bis 5, dadurch gekennzeichnet, daß als Hirudin ein recombinantes Desulfato-Hirudin (r-Hirudin) verwendet wird.

**7.** Verwendung einer Wirkstoffkombination gemäß einem oder mehreren der Ansprüche 1 bis 6 zur Herstellung von antithrombotischen Mitteln.

**8.** Verfahren zur Herstellung eines pharmazeutischen Präparates enthaltend eine nach einem der Verfahren der Ansprüche 1 bis 6 hergestellten Wirkstoffkombination, dadurch gekennzeichnet, daß man diese zusammen mit einem physiologisch unbedenklichen Träger, gegebenenfalls weiteren Hilfs- und Zusatzstoffen und gegebenenfalls weiteren therapeutisch wirksamen Stoffen in eine geeignete Darreichungsform bringt.